**Europäisches Patentamt**

**European Patent Office**

**Office européen des brevets**

(19)

(11) Veröffentlichungsnummer: **0 204 987 B1**

(12) **EUROPÄISCHE PATENTSCHRIFT**

(45) Veröffentlichungstag der Patentschrift: **21.11.91**

(51) Int. Cl.⁵: **A61K 31/355**, A61K 45/06

(21) Anmeldenummer: **86106589.4**

(22) Anmeldetag: **15.05.86**

(54) **Vitamin E-haltiges Mittel zur Verbesserung der Eigenschaften des Blutes.**

(30) Priorität: 15.05.85 DE 3517550
29.05.85 DE 3519165
20.06.85 DE 3521981
30.07.85 DE 3527193
16.08.85 DE 3529401
23.10.85 DE 3537692

(43) Veröffentlichungstag der Anmeldung:
**17.12.86 Patentblatt 86/51**

(45) Bekanntmachung des Hinweises auf die
Patenterteilung:
**21.11.91 Patentblatt 91/47**

(84) Benannte Vertragsstaaten:
**AT BE CH DE FR GB IT LI LU NL SE**

(56) Entgegenhaltungen:
**EP-A- 0 141 051        EP-A- 0 151 987**
**EP-A- 0 151 989        EP-A- 0 152 106**
**EP-A- 0 158 090        EP-A- 0 163 924**

**UNLISTED DRUGS, Band 28, Nr. 7, Juli 1976,**
**Seite 119, Chatham, N.J., US;**

**ROTE LISTE, 1984, Editio Cantor,**
**Aulendorf/Württ., DE;**

(73) Patentinhaber: **Ismail, Roshdy, Dr.**
**Siebengebirgs-Apotheke Siebengebirgsallee**
**2**
**W-5000 Köln 41 (Klettenberg)(DE)**

(72) Erfinder: **Ismail, Roshdy, Dr.**
**Siebengebirgs-Apotheke Siebengebirgsallee**
**2**
**W-5000 Köln 41 (Klettenberg)(DE)**

(74) Vertreter: **Werner, Hans-Karsten, Dr. et al**
**Deichmannhaus am Hauptbahnhof**
**W-5000 Köln 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

UNLISTED DRUGS, Band 32, Nr. 5, Mai 1980,
Seite 73, Chatham, N.J., US;

UNLISTED DRUGS, Band 34, Nr. 3, März 1982,
Seite 43, Chatham, N.J., US;

ROTE LISTE, 1980, Editio Cantor,
Aulendorf/Württ., DE;

ROTE LISTE, 1976, Editio Cantor,
Aulendorf/Württ., DE;

ROTE LISTE, 1971, Seite 168, Editio Cantor,
Aulendorf/Württ., DE;

ROTE LISTE, 1971, Seite 401, Editio Cantor,
Aulendorf/Württ., DE;

ROTE LISTE, 1961, Seite 1012, Editio Cantor,
Aulendorf/Württ., DE;

DICTIONNAIRE VIDAL, 1974, Seite 145, O.V.P.,
Paris, FR;

DICTIONNAIRE VIDAL, 1961, Seite 1824,
O.V.P., Paris, FR;

Rote Liste 1981 no. 52215. Salus.
"Herz-Schutz Kapseln"

**Beschreibung**

Die vorliegende Erfindung betrifft ein Vitamin E-haltiges Mittel zur Verbesserung der Eigenschaften des Blutes, insbesondere der Fließeigenschaften, der Immunabwehr, der Durchblutung der Peripherie der Augen, des Mittelohres, des Herzens und des Cerebrums sowie zur Behandlung abnormaler Vergrößerungen der Zellen und von Tumoren.

Vitamin E ist bekannt als Antioxidans und als Schutzvitamin für Phospholipide der Zellmembran (Lucy Ann. N.Y. Academy of Science 203, 1972, Seite 4). Es ist weiterhin bekannt, daS Vitamin E membranabdichtend wirkt (F. Mittelbach und G. Bodechtel, Münchner Medizinische Wochenschrift 110, 1968, 36, 1988 - 1993). In Tierversuchen und klinischen Tests wurde ferner nachgewiesen, daß Anämie auf Vitamin E-Mangel zurückzuführen ist. Durch Verabreichung von hohen Vitamin E-Dosen konnte eine Normalisierung der Hämolyse der Erythrozyten erreicht werden (William J. Darbey Vitamin Horm., 26 (50), Seiten 685 - 704, 1968, und Phelps DL Pediatrics 63 (6), Seiten 933 - 935, 1979). Aus den genannten Literaturstellen ist bekannt, daß durch die Verabreichung von 200 bis 800 mg Vitamin E in einem Zeitraum von 1 bis 4 Tagen die Hämolyse der Erythrozyten signifikant verbessert wird.

Vitamin E ist weiterhin zur Behandlung von Sichelzellenanämie verwendet worden (Natt CL. clin. 33, Seiten 968 - 971, 1980; Natt CL. Am. J. clin. nutr. 32, Seiten 1359 - 1362, 1979 und Gawlik G.M. Fed. Proc. 35 (3), Seite 252, 1976).

Außerdem wurde Vitamin E in einer täglichen Dosis von 750 g erfolgreich bei Thalassamie-Patienten angewendet (Kahane I ISR. J. Med. 12 (1), Seiten 11 - 15, 1976).

Vitamin E wurde auch erfolgreich eingesetzt zur Behandlung von akuter Hepatitis und alkoholischer Hepatitis (Yoshiakawa T., Takemura S., Kato H. et al., Japan J. Gastrovent, 74/7, Seiten 732 - 739, 1977). Schließlich wurden Patienten, die an Eisenmangelanämie erkrankt waren, mit Vitamin E behandelt. Hier wurde eine Verbesserung und Normalisierung des Lipidmetabolismus im Knochenmark bewirkt (Takoshi Itaga, Central Clinical Laboratory Nagasaki University of Medicine, Japan).

Es wurde nun überraschenderweise festgestellt, daß Vitamin E in Kombination mit gefäßerweiternden und/oder durchblutungsfördernden Mitteln die Eigenschaften des Blutes, insbesondere die Fließeigenschaften, verbessern kann.

Gegenstand der vorliegenden Erfindung ist somit die Verwendung von Vitamin E in Mengen von 150 bis 1000 i. E. pro Darreichungsform, gegebenenfalls in Gegenwart von durchblutungsfördernden und/oder gefäßerweiternden Mitteln, Vitamin A, Vitamin C, Vitaminen der B-Reihe, Schmerzmitteln, Antiphlogistika, Antirheumamitteln, Emulgatoren und/oder üblichen Hilfsstoffen zur Herstellung eines Arzneimittels zur Verbesserung der Eigenschaften des Blutes, wobei das Arzneimittel in Darreichungsformen aus der Gruppe Kapseln, Tabletten, Dragees und Suppositorien vorliegt. Überraschenderweise wird die Wirkung von Vitamin E in den erfindungsgemäßen Kombinationen erheblich gesteigert und dadurch die Behandlungszeit verkürzt. Aufgrund der synergistischen Eigenschaften gehen die Krankheitssymptome schneller zurück, als wenn Vitamin E allein verabreicht wird.

Aufgrund der verbesserten Eigenschaften des Blutes werden insbesondere dessen Fließeigenschaften, die Immunabwehr, die Durchblutung der Peripherie der Augen, des Mittelohres, des Herzens und des Cerebrums verbessert. Ebenso eignen sich die erfindungsgemäßen Mittel zur Behandlung abnormaler Vergrößerungen der Zellen und von Tumoren. Insbesondere lassen sich mit Hilfe der erfindungsgemäßen Mittel Schmerzen, Migräne und auch Menstruationsbeschwerden beseitigen.

Vitamin E wird in einer Konzentration von 300 bis 600 i.E. pro Darreichungsform eingesetzt. Hierbei entspricht 1 i.E. 1 mg synthetischem Vitamin E, während 1,5 i.E. 1 mg natürlichem Vitamin E gleichzusetzen sind. Vorzugsweise werden erfindungsgemäß 400 bis 600 i.E. Vitamin E pro Darreichungsform eingesetzt. Typische Kombinationspräparate enthalten 400 und 500 mg Vitamin E. Insbesondere bei Kombinationen mit Nicotinsäure und deren Derivaten werden Dosierungen von 300 bis 500 mg Vitamin E pro Darreichungsform eingesetzt. Sofern in der Vergangenheit hin und wieder geringe Mengen von nur 40 mg Vitamin E in Kombinationspräparaten zum Einsatz gekommen sind, waren diese Mittel mit Sicherheit wegen der zu niedrigen Dosierung wirkungslos, da große Teile durch die Magensäure zerstört werden und dadurch ihre Wirksamkeit verlieren (Arthur Vogelsang in Angiology 21, Seiten 275 - 279, 1970).

In einer bevorzugten Ausführungsform der vorliegenden Erfindung liegt das Arzneimittel in der Darreichungsform von Weichgelatinekapseln vor.

Vitamin E kann sowohl in Form der Ester natürlicher oder synthetischer Herkunft als auch in Form des freien Tocopherols eingesetzt werden. Für die erfindungsgemäßen Kombinationspräparate sind die durchblutungsfördernden Mittel Extr. Hippocastani, Cinnarizin, Vincamin, Pentoxyphyllin, Bamethansulfat, Peracetam, Calciumdobesilat, Weißdorn bzw. dessen Extrakt, Buflomedil, Flunarizin, Bencyclanhydrogenfumarat, Dihydroergotoxinmethansulphonat, ß-Pyridylcarbinol, Ginkoflavonglykoside, ß-Hydroxyäthylrutosid und Nicergolin geeignet. Pentoxyphyllin wird in Mengen von 150 bis 800 mg, vorzugsweise 400 bis 600 mg, verwendet. Nicotinsäure ist in Mengen von 200 bis 600 mg in Kombination mit 300 bis 600 i.E., vorzugsweise 400 bis 500 i.E. Vitamin E in den erfindungsgemäßen Präparaten enthalten. Neben den oben aufgezählten durchblutungsfördernden und gefäßerweiternden Mitteln können auch andere gleichartige Produkte verwendet werden. So sind als gefäßerweiternde Mittel Lokalanästhetika wie Procain oder Procainhydrochlorid geeignet.

Weiterhin können die erfindungsgemäßen Präparate Schmerzmittel, Antiphlogistika und/oder Antirheumamittel enthalten. Als Schmerzmittel können zum Beispiel Acetylsalicylsäure, Dichlofenac, Pyrazolon und dessen Derivate, Phenacetin, Paracetamol und dessen Derivate eingesetzt werden.

Neben Vitamin E können auch noch weitere Vitamine zugesetzt werden. Insbesondere können die erfindungsgemäßen Präparate Vitamin A und Vitamin C sowie die Vitamine der B-Reihe enthalten. Vitamin A kann in Form von Vitamin A-Palmitat, Vitamin A-Acetat sowie weiterer Ester des Vitamin A oder in Form des Betacarotin verwendet werden. Vitamin A soll in solchen Dosen zugefügt werden, daß die maximale Tagesdosis von 50000 i.E. nicht überschritten wird. Das heißt, wenn zwei Darreichungsformen pro Tag verabreicht werden sollen, muß die Dosierung zwischen 15000 und 25000 i.E. pro Darreichungsform liegen. Die erfindungsgemäßen Kombinationen mit Vitamin A und E verbessern insbesondere die Durchblutung der Extremitäten, der Peripherie des Auges, des Innenohres und des Cerebrums.

Die Wirksamkeit von Vitamin A und E bei diesen Indikationen ist besonders überraschend und eröffnet weitere neue Anwendungsgebiete für diese Vitamine.

Die erfindungsgemäßen Mittel enthalten außer den Wirkstoffen und Vitamin E übliche Träger- und Hilfsstoffe.

Ferner werden den erfindungsgemäßen Präparaten Emulgatoren zugesetzt. Überraschend wurde festgestellt, daß schon geringe Mengen von 1% Emulgator ausreichen, um die Klumpenbildung zu verhindern. Die Wirkstoffe werden besser im wässrigen Medium dispergiert bzw. suspendiert. Dies hat den Vorteil, daß die Absorption durch den Darm erleichtert wird. Eine größere Menge Emulgator ist demzufolge nicht notwendig. In der Regel reichen 0,1 bis 5% Emulgator aus, um die Klumpenbildung zu verhindern. Man kann auch bis zu 10% Emulgator zusetzen. Hierbei besteht jedoch die Gefahr, daß durch die Zugabe derartiger Mengen von Hilfsstoffen Nebenwirkungen auftreten, insbesondere, wenn das Medikament längere Zeit eingenommen wird.

Es können die üblichen Emulgatoren zugesetzt werden. Insbesondere sind Tween® 20, Chremophore, aliphatische Alkohole und partialveresterte Triglyceride geeignet. Für die vorliegende Erfindung werden Tween® 20 und Cetiol bevorzugt. Insbesondere bei diesen beiden Emulgatoren wurde beobachtet, daß durch Zugabe von 10% Emulgator die Emulgierung nicht wesentlich verbessert wird gegenüber dem Zusatz von 5% Emulgator.

Als Emulgator läßt sich erfindungsgemäß ferner Lecithin in Konzentrationen von 1 bis 20% verwenden. Hierdurch wird vor allem die Resorption von Vitamin A und E begünstigt.

Durch den Zusatz von etwa 1% herkömmlicher Emulgatoren, zum Beispiel Tween® 80, wird die Mischbarkeit von Lecithin mit den beiden oben genannten Vitaminen begünstigt und eine Klumpenbildung verhindert. Besonders vorteilhaft für die Resorption ist die Verabreichung einer Kombination von 1% Tween® mit 1 bis 20% Lecithin. Ebenso können 1% Cetiol, Oleylsäureester oder Chremophore verwendet werden. Als Lecithinpräparat wird das Sojalecithin bevorzugt.

Lecithin läßt sich auch in großen Mengen bis zu 70% verwenden. In dem Bereich von 20 bis 70% fungiert Lecithin jedoch nicht als Emulgator, sondern als Wirkstoff.

Da Vitamin E bei üblichen Temperaturen flüssig ist, bietet sich als Applikationsform insbesondere die Weichgelatinekapsel an. Die übrigen Wirkstoffe werden in Vitamin E sowie gewünschtenfalls in einem dünnflüssigen Neutralöl und einem Lösungsmittel in an sich bekannter Weise in die Weichgelatinekapseln eingebracht. Vitamin E kann auch in fester Form, zum Beispiel als Vitamin E-Succinat, in Hartgelatinekapseln oder in Tabletten- bzw. Drageeform verabreicht werden. Erfolge wurden auch bei der Behandlung mit Suppositorien erzielt, die Vitamin E enthalten. Für die Herstellung der Suppositorien können übliche Hilfs- und Trägerstoffe verwendet werden. Als schmerzstillende Mittel werden insbesondere Indometacin, Dichlofenac, Flufenaminsäure, Ibuprofen, Mefenaminsäure, Niflusminsäure und Tiaprofensäure verwendet. Der Vorteil der Verwendung von Suppositorien liegt darin, daß Vitamin E nicht durch die Magensäure zerstört werden kann.

Die erfindungsgemäßen Präparate können auch in Form von Lösungen eingesetzt werden.

Insbesondere kommen alkoholische Lösungen oder andere geeignete Lösungsmittel in Betracht. In diesem Fall sind die erfindungsgemäßen Mittel für Injektionen geeignet. Ebenso lassen sich die Präparate in Form von Tropfen verabreichen.

Durch die synergistische Wirkung der erfindungsgemäßen Präparate wird vor allen Dingen die Funktion der Zellen erheblich gesteigert und die Zellmembran abgedichtet. Diese Wirkung führt zur Heilung von verschiedenen Krankheiten. Hierzu zählen insbesondere Krankheiten, die auf einen Mangel an Durchblutung und auf Defekten der Zellen beruhen. So wird beispielsweise durch Verabreichung der erfindungsgemäßen Präparate das Immunsystem der Zellen verbessert. Infolgedessen können mit den erfindungsgemäßen Präparaten auch Krankheiten behandelt werden, die auf einer Immunschwäche beruhen. Insbesondere eignen sich die erfindungsgemäßen Mittel zur vorbeugenden Behandlung von Immunschwächekrankheiten wie Aids und dergleichen. Ebenso kann verschiedenen Infektionskrankheiten, zum Beispiel Erkältungen, vorgebeugt werden.

Die erfindungsgemäßen Kombinationen verbessern ferner die Durchblutung der Arterien, zum Beispiel die Durchblutung der Extremitäten, der Peripherie des Auges, des Innenohres und des Cerebrums. Wenn man den erfindungsgemäßen Präparaten außerdem Dimethylaminoethanol und dessen Derivate oder Salze zusetzt, werden die Durchblutung des Gehirns, die Stimulation des Zentralnervensystems und das Konzentrationsvermögen gesteigert. Auch diese Wirkung des Vitamin E in den erfindungsgemäßen Kombinationspräparaten ist überraschend und ermöglicht völlig neue Anwendungsgebiete für Vitamin E-Präparate. Hierzu zählen insbesondere Gefäßerkrankungen, wie Entzündungen der Venen (Thrombophlebitis, Varikophlebitis, Thrombophlebitis migrans, Thrombophlebitis saltans).

Ferner können die erfindungsgemäßen Mittel für die postoperative Thromboembolie-Prophylaxe eingesetzt werden. Hierbei erreicht man eine Verminderung postoperativer Thrombosen und Embolien, insbesondere in der Kombination mit Low-dose-Heparin. Außerdem kann mit Hilfe der erfindungsgemäßen Präparate der Spontanverlauf bzw. die Progression arterieller Verschlußkrankheiten beeinflußt werden. Auf diese Weise kann Rezidiven nach perkutaner Kathederrekanalisation und nach gefäßchirurgischen Eingriffen vorgebeugt werden.

Ein weiteres Einsatzgebiet der erfindungsgemäßen Präparate ist der zerebrale Gefäßverschluß (Hirninfarkt). Aus diesem Grunde eignen sich die Mittel für die Schlaganfall-Prophylaxe durch Behandlung der pathognomischen (kennzeichnenden) Vorläuferstadien der TIA (transitorisch ischämischen Attacken = fluchtige neurologische Ausfälle auf einer Körperseite) bzw. PRIND (prolongierte ischämisch bedingte neurologische Defizite = verlängerte vorübergehende neurologische Ausfälle). Daneben können die erfindungsgemäßen Mittel auch zur Prophylaxe des Herzinfarktes eingesetzt werden. Hier beruht die Wirkung der Mittel vor allen Dingen auf der Verminderung der Gefahr weiterer Koronarthrombosen nach überstandenem Herzinfarkt.

Nicht zuletzt können die erfindungsgemäßen Produkte insbesondere in ihrer Kombination mit antiphlogistischen und schmerzstillenden Mitteln zur Verminderung starker Schmerzen verwendet werden. Als schmerzstillende Mittel kommen hier insbesondere Acetylsalicylsäure, Phenacetin, Propyphenanzon, Indometacin und Dichlofenac in Frage. Bei der Herstellung von Kapseln kann Acetylsalicylsäure zum Beispiel in mikroverkapselter Form verwendet werden.

Als Füllungs- bzw. Lösungsvermittler können aber auch neutrale Öle, zum Beispiel Sojaöl oder Fettsäureglyceride, Fettalkohole, Fettsäure, Ester etc. verwendet werden. Hierdurch wird eine verzögerte Freisetzung der Salicylsäure verursacht, so daß die Mikroverkapselung der Acetylsalicylsäure entbehrlich ist.

Durch die folgenden Beispiele werden die Wirkungen der erfindungsgemäßen Präparate näher erläutert:

Beispiel 1

In einem Versuch wurde untersucht, inwieweit die Verabreichung von Vitamin E in Kombination mit durchblutungsfördernden Mitteln eine Verbesserung der Eigenschaften des Blutes mit sich bringt gegenüber der Verabreichung von reinem Vitamin E.

2 Kapseln enthaltend 400 i.E. Vitamin E (Ergebnisse: Tabelle 1) bzw. 400 i.E. Vitamin E und 10000 i.E. Vitamin A (Ergebnisse: Tabelle 1a) bzw. 200 mg Troxerutin + 400 i.E. Vitamin E + 10000 i.E. Vitamin A (Ergebnisse: Tabelle 2) wurden 2 Wochen lang 10 gesunden Probanden verabreicht. Aus den Tabellen 1, 1a und 2 sind die Ergebnisse ersichtlich. Insbesondere zeigen 5 Parameter eine signifikante Verbesserung:

1. Blutviskosität bei nativem Hämatokrit bei 0,7 l/s Schergeschwindigkeit ($p = 0,05$)
2. Blutviskosität bei Standard-Hämatokrit bei 0,7 l/s ($p = 0,001$)
3. Blutviskosität bei Standard-Hämatokrit bei 2,4 l/s ($p = 0,05$)
4. Erythrozyten-Flexibilität ($p = 0,001$)
5. Low-Densitiy-Lipoprotein ($p = 0,02$).

Hämatokrit und kolloidosmotischer Druck blieben unverändert. Das heißt, die rheologischen Effekte reflektieren eine qualitative Veränderung der Zellmembran (Flexibilität und Adhesivität = Low-sheat-Viskosität).

5

Die Erythrozyten-Flexibilität wird auch durch die alleinige Verabreichung von Vitamin E erhöht (vgl. Tabelle 1), während nur in Kombination mit dem durchblutungsfördernden Mittel ß-hydroxyrutosid 5 weitere Parameter des Blutes verbessert werden (vgl. Tabelle 2). Die Rolle der Erythrozyten-Flexibilität in der Gewebeperfusion läßt sich wie folgt erklären: Rigide Erythrozyten blockieren die Kapillaren und verursachen eine funktionelle Shunt-Strömung. Die Erythrozyten-Adhesivität in der Gewebedurchblutung hat folgende Funktion: Die verstärkte Adhesion der Blutzellen fördert die Aggregation in den postkapillaren Gefäßen, so daß der postkapillare Widerstand steigt. In solchen Fällen gibt es fast immer eine nachweisbare verstärkte Adhesivität von Thrombozyten und Leukozyten.

Die Ergebnisse der oben beschriebenen Versuche sind in den folgenden Tabellen 1, 1a und 2 zusammengestellt.

<u>Tabelle 1</u>   Mittelwerte, Standardabweichung, Signifikanz der Ergebnisse

(Vitamin E ohne durchblutungsfördernde Mittel)
Dosierung: über 2 Wochen täglich 2 Kapseln mit einem Gehalt von 400 i.E. Vitamin E

| Parameter | Dimension | Ausgangswerte | nach 2 Wochen | nach 4 Wochen | Signifikanz | | |
|---|---|---|---|---|---|---|---|
| | | 0 | 2 | 4 | 0 - 2 | 0 - 4 | 2 - 4 |
| BV bei 95 $s^{-1}$ | mPa s | 4,9± 0,6 | 4,9± 0,4 | 5,0± 0,4 | - | - | - |
| BV bei 2,4 $s^{-1}$ | mPa s | 14,9± 3,6 | 15,2± 2,3 | 15,9± 2,7 | - | - | - |
| BV bei 0,7 $s^{-1}$ | mPa s | 24,5± 7,3 | 24,5± 4,9 | 26,4± 5,5 | - | - | - |
| Plasma Viskosität | mPa s | 1,14±0,07 | 1,14+0,03 | 1,15±0,04 | - | - | - |
| Ery. Flexibilität | keine | 0,60±0,19 | 0,63±0,20 | 0,63±0,20 | 0,10 | 0,05 | - |
| Ery. Aggregation | keine | 6,5± 3,9 | 6,6± 4,1 | 6,4± 3,4 | - | - | - |
| kolloidonk. Druck | mmHg | 25,3± 2,7 | 27,0±.2,3 | 26,0± 2,2 | | | |
| Leukozyten | $x10^3$ | 6,1± 1,5 | 5,9± 1,3 | 5,8± 1,4 | - | - | - |
| Hämatokrit | % | 44± 4 | 44± 3 | 45± 3 | - | - | - |
| Hämoglobin | g% | 16,5± 1,9 | 16,9± 1,2 | 16,2± 1,1 | - | - | - |
| Erythrozyten | $x10^6$ | 5,39± 0,81 | 5,28± 0,64 | 5,30± 0,51 | - | - | - |
| Serum Tocopherol | µg/ml | 7± 2 | 14+ 7 | 14+ 7 | 0,01 | 0,01 | - |
| Serum Ges. Lipide | mg/dl | 874+186 | 909+104 | 881+155 | - | - | - |

BV = Blutviskosität

- = keine Signifikanz

EP 0 204 987 B1

## Tabelle 1a

Dosierung: über 2 Wochen täglich 2 Kapseln mit einem Gehalt von 400 mg Vitamin E + 10000 i.E. Vitamin A

Statistik: multiple Vergleiche nach Wilcoxon und Wilcox (Seite 426-429 in Sachs, L. "Angewandte Statistik" Springer, Berlin, 1984.).

| Parameter | Mittelwert ± Standardabweichung | | | Statistik (p< ...) | | |
|---|---|---|---|---|---|---|
| | Base | nach 2 W. | nach 4 W. | B-2 | B-4 | 2-4 |
| VBV bei 95 s$^{-1}$ | 4,92±0,58 | 4,96±0,58 | 4,92±0,58 | - | - | - |
| VBV bei 2,4 s$^{-1}$ | 15,6±3,0 | 15,8±3,3 | 15,8±3,0 | - | - | - |
| VBV bei 0,7 s$^{-1}$ | 26,7±6,3 | 27,2±6,4 | 27,3±6,0 | - | - | - |
| Plasma Viskosität | 1,15±0,07 | 1,17±0,06 | 1,14±0,05 | - | - | - |
| Ery. Flexibilität | 61±5 | 64±5 | 64±5 | 0,05 | 0,01 | - |
| Ery. Aggregation | 7,1±3,5 | 7,5±2,5 | 8,1±4,1 | - | - | - |
| Koll. Onk. Dr. | 26,1±2,7 | 26,2±2,0 | 25,4±1,8 | - | - | - |
| Leukozyten | 6,5±1,8 | 6,2±1,4 | 5,8±1,0 | - | - | - |
| Hämatokrit | 46,0±3,0 | 45,0±4,6 | 45,6±3,0 | - | - | - |
| Hämoglobin | 170±15 | 169±25 | 169±21 | - | - | - |
| Erythrozyten | 5,31±0,42 | 5,31±0,74 | 5,32±0,58 | - | - | - |
| Serum Tocopherol | 7.7 ± 2.5 | 16.8 ± 3.0 | 18.5 ± 1.2 | | | |
| BKS 1 St. | 2,7±1,8 | 2,8±2,0 | 2,5±0,9 | - | - | - |
| BKS 2 St. | 8,0±4,1 | 7,4±5,0 | 6,6±2,8 | - | - | - |
| BV(45%); 95 s$^{-1}$ | 4,75±0,27 | 4,85±0,35 | 4,81±0,24 | - | - | - |
| BV(45%); 2,4 s$^{-1}$ | 15,6±1,1 | 15,8±2,2 | 15,8±1,2 | - | - | - |
| BV(45%); 0,7 s$^{-1}$ | 24,6±3,2 | 26,0±5,3 | 25,9±3,0 | - | - | - |
| Triglyc. (mg/dL) | 145±44 | 158±28 | 143±28 | 0,05 | - | - |
| Cholest. (mg/dL) | 200±56 | 230±42 | 202±57 | - | - | - |
| LDL (mg/dL) | 136±20 | 130±25 | 136±23 | - | - | - |
| A (I.E/ml) | 1.2 ± 0.2 | 2.3 ± 0.1 | 3.4 ± 0.2 | | | |
| Gesamtlipide (mg/dl) | 750 ± 163 | 805 ± 124 | 820 ± 121 | | | |

Tabelle 2

Dosierung: über 2 Wochen täglich 2 Kapseln mit einem Gehalt von 200 mg Troxerutin + 400 i.E. Vitamin E + 10000 i.E. Vitamin A

| PARAMETER | | BASELINE | NACH 2 WOCHEN |
|---|---|---|---|
| Vollblut | 0,7 l/s | 21,2±3,7 | 19,2±5,1 * |
| viskosität | 2,4 l/s | 12,5±1,8 | 12,3±2,3 |
| in mPas | 94,5 l/s | 4,8±0,5 | 4,8±0,6 |
| Blutvisk. | 0,7 l/s | 23,7±1,1 | 21,1±2,3 **** |
| bei 45% Hk | 2,4 l/s | 13,7±0,6 | 13,4±0,8 * |
| in mPas | 94,5 l/s | 5,1±0,2 | 5,1±0,3 |
| Plasma visk. (mPas) | | 1,23±0,06 | 1,22±0,07 |
| Ery. Flexibilität | | 54±5 | 63±5 **** |
| Ery. Aggregation | | 10,0±3,3 | 10,0±3,3 |
| Plasma C.O.D.(mmHg) | | 25,1±1,6 | 25,3±1,7 |
| Leukozyten ($10^3$/ul) | | 5,7±1,2 | 5,7±1,2 |
| Hämatokrit (%) | | 43,1±2,2 | 42,7±2,5 |
| Hämoglobin (g/l) | | 159±10 | 156± 7 |
| Erythrozyten ($10^6$/ul) | | 5,0±0,2 | 5,0±0,3 |
| Triglyceride (mg/dl) | | 123±38 | 123±38 |
| Cholesterin (mg/dl) | | 145±30 | 144±30 |
| LDL (mg/dl) | | 118±23 | 98±31 ** |

Student t-Test: *=p<0,05; **=p<0,02; ****=p<0,001

Tabelle 3 zeigt die Ergebnisse einer Studie, bei der 200 mg Troxerutin allein verabreicht wurden. Hierbei wurden 2 Wochen lang 2 Kapseln mit je 200 mg Troxerutin, und zwar eine Kapsel nach dem Frühstück und eine Kapsel nachmittags, 10 gesunden Probanden verabreicht. Hierbei war eine gewisse Senkung der Blutviskosität und der Lipidwerte nach 2 Wochen zu beobachten. Aber nur die Veränderung der Blutviskosität bei Standardhämatokrit bei 0,7 l/s (p = 0,01) ist statistisch signifikant.

TABELLE 3

Troxerutin Studie

10 Probanden; 2x200 mg/Tag

| P A R A M E T E R | BASELINE | NACH 2 WOCHEN |
|---|---|---|
| Vollblut 94.5 1/s | $21,5\pm5,1$ | $19,5\pm4,6$ |
| viskosität 2,4 1/s | $12,6\pm2,5$ | $12,5\pm2,4$ |
| in mPas 0.7 1/s | $4,9\pm0,6$ | $4,9\pm0,6$ |
| Blutvisk. 94.5 1/s | $24,2\pm1,2$ | $22,1\pm1,7$ * |
| bei 45% Hk 2,4 1/s | $13,9\pm0,7$ | $13,7\pm0,7$ |
| in mPas 0.7 1/s | $5,2\pm0,3$ | $5,2\pm0,3$ |
| Plasma visk. (mPas) | $1,25\pm0,06$ | $1,26\pm0,06$ |
| Ery. Flexibilität | $55\pm7$ | $55\pm8$ |
| Ery. Aggregation | $9,1\pm3,2$ | $9,1\pm3,3$ |
| Plasma C.O.D.(mmHg) | $25,2\pm1,1$ | $25,2\pm1,1$ |
| Leukozyten ($10^3$/ul) | $5,5\pm1,2$ | $5,6\pm1,0$ |
| Hämatokrit (%) | $42,9\pm2,9$ | $42,7\pm3,0$ |
| Hämoglobin (g/l) | $158\pm12$ | $156\pm11$ |
| Erythrozyten ($10^6$/ul) | $4,9\pm0,4$ | $4,7\pm0,3$ |
| Triglyceride (mg/dl) | $127\pm38$ | $122\pm39$ |
| Cholesterin (mg/dl) | $157\pm24$ | $149\pm30$ |
| LDL (mg/dl) | $126\pm16$ | $119\pm31$ |

Student t-test: *$p<0,05$; **$p<0,02$; ***$p<0,01$

In einem weiteren Versuch wurden Kapseln getestet, die 400 mg Vitamin E, 10000 i.E. Vitamin A und 150 mg Extr. Hippocastani enthielten. 2 Kapseln pro Tag wurden 2 Wochen lang 10 gesunden Probanden verabreicht. Aus Tabelle 4 ist ersichtlich, daß 4 Parameter eine statistisch signifikante Verbesserung aufwiesen:

1. Blutviskosität bei standard Hämatokrit bei 0,7 l/s ($p = 0,01$)
2. Plasmaviskosität ($p = 0,05$)
3. Erythrozytenflexibilität ($p = 0,02$)

4. Gesamtcholesterin im Serum (p = 0,05).

TABELLE 4

E + A + HYPPOCASTANIE STUDIE RESULTATE

10 000 IE VIT. A + 400 mg VIT. E + 150 mg EXTR. HYPPOCAST.
Täglich 2 Kaps. über 2 Wochen

| PARAMETER | BASELINE | NACH 2 WOCHEN |
|---|---|---|
| Vollblut 0,7 1/s | 24,3$\pm$4,9 | 22,9$\pm$5,8 |
| Viskosität 2,4 1/s | 14,0$\pm$2,3 | 14,1$\pm$2,6 |
| in mPas 94,5 1/s | 5,1$\pm$0,6 | 5,3$\pm$0,6 |
| Blutvisk. 0,7 1/s | 24,1$\pm$1,2 | 22,0$\pm$1,8 *** |
| bei 45% Hk 2,4 1/s | 13,9$\pm$0,7 | 13,7$\pm$0,9 |
| in mPas 94,5 1/s | 5,1$\pm$0,2 | 5,2$\pm$0,3 |
| Plasma visk. (mPas) | 1,25$\pm$0,06 | 1,23$\pm$0,06 * |
| Ery. Flexibilität | 59$\pm$6 | 63$\pm$8 ** |
| Ery. Aggregation | 10,8$\pm$3,5 | 10,6$\pm$3,6 |
| Plasma C.O.D.(mmHg) | 24,8$\pm$1,4 | 25,0$\pm$1,8 |
| Leukozyten ($10^3$/ul) | 5,6$\pm$1,0 | 5,4$\pm$1,1 |
| Hämatokrit (%) | 44,9$\pm$3,0 | 44,7$\pm$3,7 |
| Hämoglobin (g/1) | 167$\pm$12 | 166$\pm$18 |
| Erythrozyten ($10^6$/ul) | 5,2$\pm$0,4 | 5,3$\pm$0,5 |
| Triglyceride (mg/dl) | 129$\pm$31 | 134$\pm$34 |
| Cholesterin (mg/dl) | 140$\pm$32 | 140$\pm$30 |
| LDL (mg/dl) | 111$\pm$25 | 106$\pm$29 |

Student t-Test: *=p<0,05; **=p<0,02; ***=p<0,01

2 Kapseln, die Venostasin retard enthielten, wurden zum Vergleich 10 Probanden für 14 Tage verabreicht. Venostasin retard enthält 300 mg Extr. Hippocastani und 50 mg Aescin, während die erfindungsgemäßen Präparate 10000 i.E. Vitamin A, 400 mg Vitamin E, 150 mg Extr. Hippocastani und 24 mg Aescin enthielten. Aus Tabelle 5 ist ersichtlich, daß Venostasin retard allein keine hämorheologischen

Eigenschaften aufweist.

TABELLE 5

EXTR. HIPPOCASTANI STUDIE
10 Probanden; 2 x 1 Venostasin retard pro Tag
(300 mg Extr. Hippocastani, 50 mg Aescin)

| PARAMETER | | BASELINE | NACH 2 WOCHEN |
|---|---|---|---|
| Vollblut | 0,7 1/s | $23,2 \pm 4,2$ | $24,0 \pm 5,5$ |
| viskosität | 2,4 1/s | $13,5 \pm 2,1$ | $13,8 \pm 2,7$ |
| in mPas | 94,5 1/s | $5,1 \pm 0,5$ | $5,1 \pm 0,7$ |
| Blutvisk. | 0,7 1/s | $24,2 \pm 1,2$ | $24,2 \pm 1,3$ |
| bei 45% Hk | 2,4 1/s | $13,9 \pm 0,7$ | $14,0 \pm 0,7$ |
| in mPas | 94,5 1/s | $5,2 \pm 0,3$ | $5,2 \pm 0,3$ |
| Plasma visk. (mPas) | | $1,25 \pm 0,06$ | $1,26 \pm 0,07$ |
| Ery. Flexibilität | | $54 \pm 5$ | $54 \pm 6$ |
| Ery. Aggregation | | $8,6 \pm 2,4$ | $8,5 \pm 2,1$ |
| Plasma C.O.D.(mmHg) | | $24,8 \pm 1,3$ | $25,1 \pm 1,4$ |
| Leukozyten ($10^3$/ul) | | $5,5 \pm 1,2$ | $5,6 \pm 1,2$ |
| Hämatokrit (%) | | $44,2 \pm 2,6$ | $44,5 \pm 2,9$ |
| Hämoglobin (g/1) | | $164 \pm 11$ | $164 \pm 12$ |
| Erythrozyten ($10^6$/ul) | | $5,1 \pm 0,3$ | $5,1 \pm 0,4$ |
| Triglyceride (mg/dl) | | $140 \pm 29$ | $135 \pm 32$ |
| Cholesterin (mg/dl) | | $152 \pm 41$ | $148 \pm 40$ |
| LDL (mg/dl) | | $106 \pm 31$ | $110 \pm 37$ |

Student t-test: keine significante Veränderung

In einem weiteren Versuch wurden Kapseln verabreicht, die 400 mg Vitamin E und 100 mg Benzyclanfumarat enthielten. Aus den Tabellen 6 und 7, die die gemessenen Blutparameter der Testpersonen vor (Tabelle 6) und nach (Tabelle 7) der Untersuchung zeigen, ist ersichtlich, daß die Spontanaggregation der Thrombozyten bei Einnahme von 2 x 400 mg Vitamin E + 2 x 100 mg Benzyclanfumarat pro Tag in 14 Tagen signifikant abfiel. Aus "Die medizinische Welt" 25.867.869 (1974) ist bekannt, daß die Einnahme von Benzyclanfumarat allein bei Dosen von 200 bis 400 mg zu keiner thrombozytenaggregationshemmenden Wirkung führt. Dagegen wird bei Kombination von Benzyclanfumarat und Vitamin E eine signifikante Verminderung der Thrombozytenaggregation erreicht. Die Verminderung der Thrombozytenaggregation deutet auf thromboseprophylaktische Eigenschaften hin. Weiterhin fiel der Triglyceridwert von 125 ± 53,7 auf 102 ± 33 ab. Die Natriumkonzentration fiel von 141 ± 2,0 auf 128 ± 4,0 ab. Der Kaliumspiegel wurde nicht signifikant verändert. Ebenso wurden die Werte für Blutbild, Hämatokrit, Bilirubin, Kreatinin, Harnsäure, Cholesterin und Transaminasen nicht verändert. Die Ergebnisse können aus den nachfolgenden Tabellen 6 und 7 entnommen werden.

EP 0 204 987 B1

**T A B E L L E 6**

Rheologische Daten    400 mg Vitamin E + Bencyclanfumarat

(vor Beginn der Einnahme)

| | Erythrozyten-aggregation SEA 35 | | Erythrozyten-fluidität µl/s | | Plasma-viskosität mPa/s | Thrombozytenaggregation spontan | | | ADP | | | Collagen | | |
|---|---|---|---|---|---|---|---|---|---|---|---|---|---|---|
| | Akt. Hkt. | Hkt. 35% | 80% | 50% | | V | ./. | S | V | ./. | S | V | ./. | S |
| 1 | 12,2 | 9,3 | 12,0 | 5,56 | 1,29 | 7 | 3 | 21 | 3 | 3 | 19 | 3 | 7 | 19 |
| 2 | 19,3 | 14,8 | 11,49 | 4,12 | 1,34 | 3 | 8 | 15 | 74 | 48 | 207 | 80 | 75 | 270 |
| 3 | 19,8 | 13,2 | 17,60 | 9,50 | 1,31 | 5 | 5 | 14 | 6 | 3 | 17 | 2 | 3 | 15 |
| 4 | 13,1 | 13,4 | 8,20 | 4,20 | 1,29 | 3 | 9 | 17 | 4 | 12 | 8 | 4 | 9 | 21 |
| 5 | 12,9 | 14,4 | 10,40 | 5,06 | 1,27 | 8 | 5 | 40 | 9 | 1 | 33 | 2 | 2 | 25 |
| 6 | 9,4 | 9,5 | 14,52 | 7,40 | 1,24 | 9 | 3 | 46 | 4 | 4 | 37 | 2 | 10 | 28 |
| 7 | 9,5 | 6,4 | 12,20 | 7,20 | 1,22 | 2 | 6 | 14 | 5 | 15 | 18 | 3 | 11 | 31 |
| 8 | 8,3 | 6,9 | 10,62 | 6,60 | 1,22 | 7 | 2 | 34 | 17 | 9 | 81 | 6 | 4 | 28 |
| 9 | 12,2 | 12,0 | 9,25 | 5,35 | 1,28 | 12 | 4 | 43 | 7 | 4 | 41 | 26 | 9 | 80 |
| 10 | 8,0 | 7,4 | 11,60 | 6,40 | 1,18 | 10 | 8 | 43 | 7 | 4 | 36 | 18 | 5 | 35 |
| x | 12,5 | 10,7 | 11,8 | 6,13 | 1,26 | 6,6 | 5,3 | 28,7 | 8,0 | 6,1 | 32 | 7,3 | 6,7 | 46,9 |
| SD | 4,2 | 3,2 | 2,67 | 1,64 | 0,05 | 3,3 | 2,4 | 13,6 | 5,4 | 4,1 | 21,5 | 8,6 | 3,3 | 44,5 |

**TABELLE 7**

Rheologische Daten 400 mg Vitamin E + Bencyclanfumarat
(nach Abschluß der Einnahme)

| | Erythrozytenaggregation SEA 35 Akt. Hkt. 35% | Akt. Hkt. | Erythrozytenfluidität µl/s 80% | 50% | Plasmaviskosität mPa/s | spontan V | spontan ./. | spontan S | ADP V | ADP ./. | ADP S | Collagen V | Collagen ./. | Collagen S |
|---|---|---|---|---|---|---|---|---|---|---|---|---|---|---|
| 1 | 9,4 | 8,0 | 10,55 | 5,28 | 1,24 | 11 | 5 | 40 | 6 | 10 | 35 | 5 | 10 | 30 |
| 2 | 13,7 | 12,8 | 14,67 | 5,60 | 1,31 | 21 | 34 | 78 | 6 | 9 | 20 | 68 | 77 | 134 |
| 3 | 13,0 | 17,4 | 11,71 | 5,70 | 1,34 | 8 | 9 | 16 | 6 | 15 | 25 | 7 | 8 | 56 |
| 4 | 9,7 | 11,4 | 8,60 | 4,20 | 1,26 | 10 | 15 | 33 | 9 | 12 | 29 | 11 | 12 | 24 |
| 5 | 14,2 | 12,5 | 14,31 | 7,37 | 1,26 | 14 | 5 | 58 | 14 | 4 | 52 | 9 | 4 | 38 |
| 6 | 12,2 | 12,7 | 8,5 | 4,55 | 1,23 | 12 | 5 | 64 | 14 | 6 | 48 | 6 | 30 | 5 |
| 7 | 6,9 | 9,4 | 17,7 | 8,93 | 1,22 | 12 | 6 | 59 | 6 | 4 | 41 | 6 | 12 | 33 |
| 8 | 6,8 | 8,9 | 14,02 | 7,40 | 1,24 | 5 | 13 | 25 | 6 | 16 | 16 | 10 | 23 | 16 |
| 9 | 11,1 | 11,9 | 7,23 | 3,50 | 1,19 | (65) | 4 | 28 | 6 | 6 | 15 | 10 | 1 | 58 |
| 10 | 7,0 | 8,0 | 7,37 | 3,60 | 1,15 | 10 | 6 | 40 | 7 | 11 | 40 | 6 | 13 | 21 |
| x | 9,7 | 10,6 | 11,46 | 5,6 | 1,24 | 11,4 | 7,4 | 44 | 8 | 9,3 | 32,1 | 7,8 | 12,6 | 31,2 |
| SD | 4,1 | 4,3 | 3,6 | 1,8 | 0,05 | 4,4 | 4,0 | 19,8 | 3,3 | 4,3 | 13,1 | 2,2 | 9,0 | 17,5 |

In einem weiteren Versuch wurde die Wirkung einer Kombination untersucht, die aus 500 mg Vitamin E und 300 mg Lecithin bestand. Die Ergebnisse sind in Tabelle 8 zusammengestellt. Hierbei Zeigt sich, daß Vitamin E kombiniert mit Lecithin eine starke hämorheologische Wirkung aufweist. Dies ist aus der Veränderung der Plasmaviskosität und der Vollblutviskosität bei niedrigen Schergeschwindigkeiten zu ersehen. Signifikant ist auch die Absenkung des LDL-Spiegels. Dagegen veränderte sich die Erythrozyten-Flexibilität bei diesem Versuch nicht.

TABELLE 8

VITAMIN E + LECITHIN STUDIE

10 Probanden; 2 x (500 mg Vit. E + 300 mg Lecithin) / Tag

| P A R A M E T E R | | BASELINE | NACH 2 WOCHEN |
|---|---|---|---|
| Vollblut | 0,7 1/s | $21,6 \pm 4,1$ | $19,8 \pm 2,4$ * |
| viskosität | 2,4 1/s | $12,7 \pm 2,0$ | $12,4 \pm 1,3$ |
| in mPas | 94,5 1/s | $4,9 \pm 0,5$ | $4,9 \pm 0,4$ |
| Blutvisk. | 0,7 1/s | $24,1 \pm 1,5$ | $21,9 \pm 1,2$ *** |
| bei 45% Hk | 2,4 1/s | $13,9 \pm 0,9$ | $13,4 \pm 0,9$ ** |
| in mPas | 94,5 1/s | $5,2 \pm 0,3$ | $5,1 \pm 0,3$ |
| Plasma visk. (mPas) | | $1,24 \pm 0,07$ | $1,22 \pm 0,06$ ** |
| Ery. Flexibilität | | $57 \pm 6$ | $58 \pm 5$ |
| Ery. Aggregation | | $9,5 \pm 3,5$ | $9,4 \pm 3,4$ |
| Plasma C.O.D.(mmHg) | | $25,7 \pm 1,3$ | $25,6 \pm 1,1$ |
| Leukozyten ($10^3$/ul) | | $5,5 \pm 1,3$ | $5,5 \pm 1,2$ |
| Hämatokrit (%) | | $43,1 \pm 2,8$ | $43,1 \pm 2,1$ |
| Hämoglobin (g/l) | | $160 \pm 10$ | $159 \pm 8$ |
| Erythrozyten ($10^6$/ul) | | $5,0 \pm 0,4$ | $5,1 \pm 0,2$ |
| Triglyceride (mg/dl) | | $138 \pm 32$ | $132 \pm 35$ |
| Cholesterin (mg/dl) | | $141 \pm 24$ | $135 \pm 21$ |
| LDL (mg/dl) | | $105 \pm 28$ | $90 \pm 16$ * |

Student t-test: *p<0,05; **p<0,02; ***p<0,01

Weitere Beispiele der erfindungsgemäß anwendbaren Präparate sind in den folgenden Beispielen zusammengestellt:

## BEISPIEL 2

Kapsel enthaltend
250 mg Nicotinsäure
400 mg D,L-alpha-Tocopherolacetat;
150 mg Sojabohnenöl;

## BEISPIEL 3

Kapsel enthaltend
200 mg ß-Hydroxyäthyl-rutoside;
300 mg D-alpha-Tocopherolacetat;
180 mg Sojaöl;

## BEISPIEL 4

wie Beispiel 3,
jedoch mit 400 mg D-alpha-Tocopherolacetat anstelle von 300 mg

## BEISPIEL 5

Kapsel enthaltend
150 mg Extract Hippocastani (enthalten 25 mg Aescin);
300 mg D-alpha-Tocopherol;
150 mg Sojaöl;
(und wird am besten 2 x 2 Kapseln täglich eingenommen)

## BEISPIEL 6

Kapsel enthaltend
300 mg Xantinolnicotinat;
400 mg D-alpha-Tocopherol;
190 mg Sojaöl;

## BEISPIEL 7

Kapsel enthaltend
150 mg Extract Hippocastani (enthalten 25 mg Aescin);
250 mg Vitamin E;
150 mg Sojaöl;
(am besten werden 2 x 2 Kapseln täglich eingenommen)

## BEISPIEL 8

Kapsel enthaltend
5 mg Vitamin $B_1$;
5 mg Vitamin $B_2$;
5 mg Vitamin $B_6$;
200 mg ß-Hydroxyäthyl-rutoside;
300 mg Vitamin E;
50 mg Nicotinsäureamid;
200 mg Sojaöl;

## BEISPIEL 9

gemäß Beispiel 8,
jedoch mit 400 mg Vitamin E anstelle von 300 mg. Es wird hier empfohlen, 2 x 1 Kapsel/Tag (1 nach dem Frühstück und 1 nachmittags) einzunehmen.

## BEISPIEL 10

Kapsel enthaltend
100 mg Nicotinsäure;
100 mg Roßkastanienextract (enthalten 16 mg Aescin);
300 mg D-alpha-Tocopherolacetat;
200 mg Sojaöl;
(hier wird bevorzugt 2 x 2 Kapseln täglich)

**BEISPIEL 11**

Kapsel enthaltend
200 mg Inositol-Nicotinat;
300 mg D-alpha-Tocopherol-Konzentrat;
150 mg Sojaöl;
(es wird hier 3 x 1 Kapsel täglich empfohlen)

**BEISPIEL 12**

Kapsel enthaltend
50 mg Procainhydrochlorid;
400 mg D-alpha-Tocopherol-Konzentrat;
150 mg Sojaöl;

**BEISPIEL 13:**

Kapsel enthaltend
50 mg Procainhydrochlorid;
400 mg D,L-alpha-Tocopherolacetat;
5 mg Vitamin $B_1$;
5 mg Vitamin $B_2$;
5 mg Vitamin $B_6$;
150 mg Sojaöl oder Maisöl;

**BEISPIEL 14**

gemäß Beispiel 1,
jedoch mit 25 mg Procainhydrochlorid anstelle von 50 mg (es wird hier 2 x 1 Kapsel täglich empfohlen).

**BEISPIEL 15**

Tropfen enthaltend
100 ml 90 % Äthylalkohol enthalten:
40 g D,L-alpha-Tocopherolacetat;
4,5 g Extract Hippocastani (enthalten 750 mg Aescin);

**BEISPIEL 16**

Kapsel enthaltend
  1) 4,5 mg entsprechend Dihydroergotoxin-methan-sulphonat;
  2) 400 mg D,L-alpha-Tocopherolacetat;
  20 mg Sojaöl

**BEISPIEL 17**

Kapsel enthaltend
50 mg Procain-Hydrochlorid;
200 mg Nicotinsäure;
400 mg Vitamin E;
150 mg Maisöl;

18

### BEISPIEL 18

Kapsel enthaltend
150 mg Bencylan-hydrogenfumarat;
400 mg Vitamin E als D,L-alpha-Tocopherolacetat;
150 mg Sojaöl

### BEISPIEL 19

Kapsel enthaltend
100 mg Bencylanfumarat
400 mg DL-alpha-Tocopherolacetat
150 mg Sojaöl
(es wird 2 x 1 Kapsel täglich empfohlen)

### BEISPIEL 20

Suppositorium enthaltend
450 mg D-alpha-Tocopherol-Konzentrat
30 mg Nicotinsäurebenzylester
100 mg Diclofenac-Natrium
ad 2,0 g Stadimol

### BEISPIEL 21

wie Beispiel 20,
jedoch mit 25 mg Diclofenac anstelle von 100 mg

### BEISPIEL 22

wie Beispiel 20,
jedoch mit 50 mg Diclofenac anstelle von 100 mg.

### BEISPIEL 23

Suppositorium enthaltend
450 mg D,L-alpha-Tocopherol
20 mg Cetiol (Ölsäureoleyester)
100 mg Zinkoxid
100 mg Diclofenac Natrium
ad 2,0 g Stadimol

### BEISPIEL 24

wie Beispiel 23,
jedoch mit 25 mg bzw. 50 mg Diclofenac

### BEISPIEL 25

Suppositorium enthaltend
400 mg Vitamin E
200 mg $\beta$-Hydroxyäthylrutoside
40 mg Cetiol
ad 2,0 g Stadimol

### BEISPIEL 26

Suppositorium enthaltend

350 mg Vitamin E
150 mg Extract Hippocastani (enthält ca. 24 mg Aescin)
ad 2,o g Stadimol

**BEISPIEL 27**

Gemäß Beispiel 26 wurden Suppositorien hergestellt, jedoch mit
3oo mg Vitamin E und
200 mg Tri-äthylorutoside
50 mg Indometacin

**BEISPIEL 28**

Kapsel enthaltend

| | |
|---|---|
| Pentoxyfyllin | 400 mg |
| Vitamin E | 400 mg |
| Vitamin A Acetat | 15.000 I.E. |
| Sojaöl | 120 mg |

**BEISPIEL 29**

Kapsel enthaltend

| | |
|---|---|
| Pentoxyfyllin | 350 mg |
| Vitamin E | 350 mg |
| Sojaöl | 120 mg |

**BEISPIEL 30**

Kapsel enthaltend

| | |
|---|---|
| Naftidirofuryl-Hydrogenoxalat | 100 mg |
| Vitamin E | 500 mg |
| Sojaöl | 150 mg |

**BEISPIEL 31:**

Kapsel enthaltend

| | |
|---|---|
| Cinnarizin | 75 mg |
| Vitamin E | 400 mg |
| Vitamin A Palmitat | 15.000 I.E. |
| Vitamin $B_1$, $B_2$, $B_6$ zu gleichen Teilen | 10 mg |
| Vitamin $B_{12}$ | 5 mg |
| Sojaöl | 150 mg |

(es wird 2 x 1 Kapsel täglich empfohlen)

**BEISPIEL 32**

100 ml Tropfen aus Äthylalkohol enthaltend

| Cinnarizin | 7,5 g |
|---|---|
| Vitamin E | 4,0 g |
| Vitamin A Palmitat | 2,5 Millionen Einheiten |

**BEISPIEL 33**

Kapsel enthaltend

| Xantinolnicotinat | 250 mg |
|---|---|
| Vitamine E (DL-alpha-Tocopherol) | 400 mg |
| Tween® 80 | 80 mg |
| Sojaöl | 150 mg |

**BEISPIEL 34**

1) Kapsel oder Tablette enthaltend
Dihydroergotoxinmethansulfonat 1,5 g
aus (0,5 g Dihydroergocristinmethansulfonat)
0,5 g Dihydroergocorninmethansulfonat
0,333 mg alpha-Dihydroergocryptinmethansulfonat
0,167 mg β-Dihydroergocryptinmethansulfonat)
2) Kapsel enthaltend
400 mg Vitamin E
400 mg Lecithin + 40 mg Sojaöl

**BEISPIEL 35**

Kapsel enthaltend

| ß-Pyridyl-carbinol-tartrat entspricht 150 mg Pyridylcarbinol | 360 mg |
|---|---|
| D-alpha-Tocopherolacetat | 400 mg |
| Sojaöl | 150 mg |

**BEISPIEL 36**

Kapsel enthaltend

21

| D,L-alpha-Tocopherol | 400 mg |
|---|---|
| β-Hydroxyäthylrutosid | 300 mg |
| Vitamin A Palmitat | 15.000 I.E. |
| Sojaöl | 150 mg |

**BEISPIEL 37**

Kapsel enthaltend

| Ginkoflavonglykoside | 3,0 mg |
|---|---|
| Vitamin E D,L-alpha-Tocopherolacetat | 300 mg |
| Sojaöl | 100 mg |

**BEISPIEL 38**

Kapsel enthaltend

| Nicotinsäure | 300 mg |
|---|---|
| Vitamin E | 400 mg |
| Vitamin A Palmitat | 15.000 I.E. |
| Cetiol | 20 mg |
| Sojaöl | 150 mg |

**BEISPIEL 39**

Kapsel enthaltend

| D,L-alpha-Tocopherolacetat | 200 mg |
|---|---|
| β-Hydroxyäthylrutosid | 300 mg |
| Diclofenac Natrium | 100 mg |
| Sojaöl | 120 mg |

**BEISPIEL 40**

Kapsel enthaltend

| Dl-alpha-Tocopherolacetat | 400 mg |
|---|---|
| β-Hydroxyäthylrutosid | 200 mg |
| Diclofenac | 25 mg (50 mg) |
| Sojaöl | 120 mg |

(es wird mindestens 2 x 1 Kapsel täglich empfohlen)

**BEISPIEL 41**

Kapsel enthaltend

22

| Pentoxyfyllin | 400 mg |
|---|---|
| Vitamin E D,L-alpha-Tocopherolacetat | 400 mg |
| Tween (R) 80 | 10 mg |
| Sojaöl | 150 mg |

**BEISPIEL 42**

Kapsel enthaltend

| Bamethansulfat | 25 mg |
|---|---|
| D,L-alpha-Tocopherolacetat | 250 mg |
| Vitamin A Palmitat | 10.000 I.E. |
| Sojaöl | 150 mg |

**BEISPIEL 43**

Kapsel enthaltend

| Vincamin | 30 mg |
|---|---|
| Vitamin E D,L-alpha-Tocopherolacetat | 400 mg |
| Vitamin A Palmitat | 30.000 I.E. |
| Sojaöl | 150 mg |

**BEISPIEL 44**

Gem. Beispiel 43,
jedoch ohne Vitamin A.
(es wird hier ebenfalls 2 x 1 Kapsel täglich empfohlen)

**BEISPIEL 45**

Kapsel enthaltend
100 mg Indometacin
400 mg D,L-alpha-Tocopherolacetat
300 mg Lecithin
40 mg Sojaöl

**BEISPIEL 46**

Gem. Beispiel 45
jedoch mit 25 mg bzw. 50 mg Indometacin

**BEISPIEL 47**

23

Kapsel enthaltend

| Vitamin E D,L-alpha-Tocopherolacetat | 400 mg |
|---|---|
| Sojalecithin | 200 mg |
| Sojaöl | 120 mg |
| Tween® 80 | 8 mg |

**BEISPIEL 48:**

Kapsel enthaltend

| Dl-alpha-Tocopherolacetat | 500 mg |
|---|---|
| Sojalecithin 45 % | 300 mg |
| Sojaöl | 150 mg |

(es wird empfohlen, 2 x 1 Kapsel täglich einzunehmen)

Die Kapseln beeinflussen das Cholesterin, Lipoprotein und Fett bzw. Stoffwechsel überraschenderweise positiv, insbesondere in Gegenwart von 200 mg Troxyrutin oder anderen durchblutungsfördernden Mitteln.

**BEISPIEL 49**

Kapsel enthaltend

| Naftidirofuryl-Hydrogenoxalat | 10 mg |
|---|---|
| Vitamin E (D-alpha-Tocopherol-Konzentrat) | 500 mg |
| Sojalecithin | 25 mg |
| Sojaöl | 150 mg |

**BEISPIEL 50**

Gem. Beispiel 49,
jedoch mit 400 mg Vitamin E Dl-alpha-Tocopherolacetat und
300 mg Sojalecithin 45 %.
(es wird empfohlen, 2 x 1 Kapsel täglich zu den verschiedenen Mahlzeiten einzunehmen)

**BEISPIEL 51**

Kapsel enthaltend

| Cinnarizin | 75 mg |
|---|---|
| Vitamin E-D-alpha-Tocopherol-acetat | 400 mg |
| Vitamin $B_1$, $B_2$, $B_6$ zu gleichen Teilen | 10 mg |
| Vitamin $B_{12}$ | 5 g |
| Sojaöl | 100 mg |
| Sojalecithin | 280 mg |

BEISPIEL 52

100 ml Tropfen aus Äthylalkohol enthaltend

| Cinnarizin | 7,5 g |
| Vitamin E | 4,0 g |
| Vitamin-A-Palmitat | 2,5 Millionen Einheiten |
| Lecithin | 2,5 g |

BEISPIEL 53

Kapsel enthaltend

Xantinolnicotinat                        500 mg

Vitamin E (D,L-alpha-Tocopherol)                                  400 mg

Vitamin-A-Palmitat              25.000 I.E.

Tween (R) 80                              20 mg

Sojaöl                                   150 mg

Sojalecithin                              25 mg

BEISPIEL 54

Tropfen in 100 ml Äthylalkohol

Dihydroergotoxinmethansulphonat            1,6 g

aus

(0,5 g Dihydroergocristinmethansulfonat

0,5 Dihydroergocorninmethansulfonat)

333 mg alpha-Dihydroergocryptinmethansulfonat
167 mg ß-Dihydroergocryptinmethansulfonat)

Vitamin E (DL-alpha-Tocopherolacetat) 3,5 g

Vitamin-A-Palmitat    1,5 Millionen Einheiten

Sojalecithin                              3,5 g

BEISPIEL 55

Kapsel enthaltend

| | |
|---|---|
| ß-Pyridil-carbinol-tartrat | 360 mg |
| entspricht 150 mg Pyridylcarbinol | |
| D-alpha-Tocopherolacetat | 400 mg |
| Vitamin-A-Palmitat | 10.000 I.E. |
| Sojaöl | 100 mg |
| Sojalecithin | 150 mg |
| Tween 20 | 6 mg |

**BEISPIEL 56**

Kapsel enthaltend

| | |
|---|---|
| DL-alpha-Tocopherol | 400 mg |
| β-Hydroxyäthylrutosid | 300 mg |
| Indometacin | 50 mg |
| Sojaöl | 100 mg |
| Sojalecithin | 250 mg |

**BEISPIEL 57**

Kapsel enthaltend

| | |
|---|---|
| Ginkoflavonglykoside | 3,0 mg |
| Vitamin E DL-alpha-Tocopherolacetat | 300 mg |
| Sojaöl | 100 mg |
| Sojalecithin | 200 mg |

**BEISPIEL 58:**

Kapsel enthaltend

| | |
|---|---|
| Nicotinsäure | 300 mg |
| Vitamin E | 400 mg |
| Vitamin-A-Palmitat | 15.000 I.E. |
| Cetiol (Oleylsäureester) | 10 mg |
| Sojaöl | 100 mg |
| Sojalecithin | 50 mg |

**BEISPIEL 59**

Kapsel enthaltend

EP 0 204 987 B1

| D-alpha-Tocopherol | 200 mg |
|---|---|
| Lecithin | 500 mg |
| Sojaöl | 180 mg |
| Tween® 80 | 10 mg |

(hier wie empfohlen, eine zweite Troxirutin-Kapseln 200 mg einzunehmen - 2 x 2 Kapseln täglich -)

**BEISPIEL 60**

Kapseln wie obige beiden Beispiele,
jedoch mit D,L-alpha-Tocopherol-Acetat anstelle von D-alpha-Tocopherol.

**BEISPIEL 61**

Kapsel enthaltend

| D-alpha-Tocopherol | 400 mg |
|---|---|
| Lecithin | 400 mg |
| Sojaöl | 200 mg |
| Tween® 80 | 15 mg |

**BEISPIEL 62**

Kapsel enthaltend

| Dl-alpha-Tocopherolacetat | 400 mg |
|---|---|
| $\beta$-Hydroxyäthylrutosid | 200 mg |
| Sojalecithin 45 % | 300 mg |
| Sojaöl | 80 mg |

**BEISPIEL 63:**

Kapsel enthaltend

| Dl-alpha-Tocopherolacetat | 400 mg |
|---|---|
| Bencylanfumarat | 100 mg |
| Sojalecithin 45 % | 300 mg |
| Sojaöl | 50 mg |

Die Beispiele, die einen hohen Gehalt an Lecithin und Vitaminen haben, beeinflussen das Cholesterin und Lipid bzw. Fettstoffwechsel günstig.

**BEISPIEL 64**

Kombipackung:
Kapsel enthaltend

27

1) D-alpha-Tocopherolkonzentrat     200 mg

    Ascorbinsäure     500 mg

    Sojaöl     150 mg

    Sojalecithin     10 mg

2) Kapsel enthaltend

    Troxerutin     300 mg

    Sojaöl     150 mg

2 x 2 Kapseln täglich erhöht die Immunität gegen Infektionen und Viruserkrankungen.

**BEISPIEL 65**

Kapsel enthaltend

1) D-alpha-Tocopherolkonzentrat     400 mg

    Vitamin C     300 mg

    Sojaöl     150 mg

2) Kapsel enthaltend

    Bencylanfumarat     100 mg

    Sojaöl     180 mg

**BEISPIEL 66**

Kapsel enthaltend

1) Dl-alpha-Tocopherolacetat     300 mg

    Vitamin C     400 mg

    Sojaöl

2) Pentoxyphyllin     400 mg

    Sojaöl     200 mg

Es wird empfohlen, 2 x 2 Kapseln täglich einzunehmen. Diese Kapseln erhöhen ebenfalls die Zellen des Immunsystems gegen Infektions- und und Viruserkrankungen.

**BEISPIEL 67**

Gem. Beispiel 66,
jedoch anstelle von Pentoxyphyllin-Kapseln wurde 300 mg-Kapseln extract Hippocastani verwendet.

In allen Beispielen wurde Sojaöl zwischen 50 und 200 mg pro Kapsel zugesetzt. Es können auch andere neutrale Öle wie Olivenöl, Rüböl etc. verwendet werden.

EP 0 204 987 B1

**BEISPIEL 68**

Kombipackung enthält:

1) Kapsel oder Tablette   microverkapselt enthaltend
   Acetylsalicylsäure      400 mg
2) Vitamin E               400 mg
   Lecithin                300 mg
   Sojaöl                   50 mg

**BEISPIEL 69**

Kapsel enthaltend

| Acetylsalicylsäure | 300 mg |
|---|---|
| Vitamin E - succinat | 300 mg |

**BEISPIEL 70**

Kombipackung
Kapsel enthaltend

1) Acetylsalicylsäure        250 mg
2) Hydroxyäthylrutosid       200 mg
   Vitamin E                 400 mg
   Sojaöl                     50 mg

**BEISPIEL 71**

Kombipackung enthält:

1) Kapsel oder Tablette enthaltend
   Acetylsalicylsäure        500 mg
   Kapsel enthaltend
2) ß-Hydroxyäthylrutosid     200 mg
   Vitamin E                 400 mg
   Sojaöl                     50 mg

**BEISPIEL 72**

Kombipackung enthält:

Kapsel A enthaltend
600 mg Vitamin E
100 mg Sojabohnenöl
Kapsel B enthaltend
1,5 mg Dihydroergotoxinmethansulphat
75 mg Glykol

**BEISPIEL 73**

Kombipackung enthält:
Kapsel A enthaltend
800 mg Vitamin E
150 mg Sojaöl
Kapsel B oder Tablette enthaltend
600 mg Pentoxyphyllin in Retard Form

**BEISPIEL 74**

Kombipackung enthält:
Kapsel A enthaltend
1 g dl-alpha-Tocopherolacetat
150 mg Sojaöl
Dragees B enthaltend
300 mg Troxerutin in Retardform

**BEISPIEL 75**

Kapsel enthaltend
200 mg Vitamin E
300 mg $\beta$-Hydroxyäthylrutosid
150 mg Sojaöl

**BEISPIEL 76**

Kapsel enthaltend
400 mg Peracetam
400 mg Dl-alpha-Tocopherolacetat
150 mg Sojaöl
50 mg Sojalecithin

**BEISPIEL 77**

Kapsel enthaltend
300 mg Peracetam
400 mg D-alpha-Tocopherol Konzentrat
200 mg $\beta$-Hydroxyäthylrutosid
150 mg Sojaöl
50 mg Sojalecithin

**BEISPIEL 78**

Kombipackung enthält:
A Kapsel enthaltend
400 mg Peracetam
400 mg Vitamin E
130 mg Rüböl
40 mg Hydriertes Sojabohnenöl
10 mg Sojalecithin

EP 0 204 987 B1

B Kapsel enthaltend
1,5 mg Dihydroergotoxinmethansulfonat-Gemische
50 mg Sojaöl
100 mg Sojalecithin

**BEISPIEL 79**

Kapsel enthaltend
350 mg Peracetam
400 mg Dl-alpha-Tocopherolacetat
70 mg Cinnarizin
100 mg Sojaöl
50 mg Hydriertes Sojaöl
30 mg Sojalecithin

**BEISPIEL 80**

Kombipackung aus:
Kapsel A enthaltend
600 mg Peracetam
150 mg Sojabohnenöl
50 mg Sojalecithin
Kapsel B enthaltend
400 mg Dl-alpha-Tocopherolacetat
250 mg Nicotinsäure
100 mg Sojaöl

**BEISPIEL 81**

Kapsel enthaltend
200 mg Vitamin E
30 mg Fruct. Crataegus sicc. stand. auf palycyamidem 1,5 mg
250 mg Lecithin min. 45%
100 mg Sojaöl
Es wird hier empfohlen 3 x 2 bzw. 2 x 2 Kapseln Täglich einzunehmen

**BEISPIEL 82**

Kombipackung, Kapsel enthaltend
1. 60 mg Fruct. Crataegus sicc. stand. auf palycyamidem 3 mg
200 mg Vitamin E
100 mg Sojaöl
Kapsel enthaltend
2. 200 mg Vitamin E
500 mg Sojalecithin
100 mg Sojaöl

**BEISPIEL 83**
Kapsel enthaltend
400 mg Calciumdobisilat
400 mg Vitamin E
50 mg Sojaöl

**BEISPIEL 84**

Kapsel A enthaltend
400 mg Calciumdobisilat
400 mg Lecithin mit 45%

31

50 mg Sojaöl
Kapsel B enthaltend
200 mg Troxerutin
400 mg Vitamin E

**BEISPIEL 85**

Kombipackung enthaltend:
Kapsel A enthaltend
500 mg Calciumdobisilat
300 mg Lecithin 45%
50 mg Sojaöl
Kapsel B enthaltend
400 mg Vitamin E
200 mg Lecithin mind. 45%
50 mg Sojaöl

**BEISPIEL 86**

Kapsel A enthaltend
400 mg Peracetum
150 mg Sojaöl
50 mg Sojalecithin
Kapsel B enthaltend
400 mg DL-alpha-Tocopherolacetat
150 mg extract Hippocastani

**BEISPIEL 87**

Kapsel aus
60$^{mg}$Fructus crategus siccatum
400 mg Dl-alpha-Tocopherolacetat
150 mg extract Hippocastani
150 mg Sojaöl
10 mg Tween® 80

**BEISPIEL 88**

Kapsel aus:
60 mg Fructus Crataegus siccatum stand. auf palycyasidem 3 mg
400 mg Dl-alpha-Tocopherolacetat
150 mg Sojaöl
50 mg Sojalecithin (2 x 1 Kapsel täglich)

**BEISPIEL 89**

Kapsel aus
60 mg Fruct. Crataegus sicc. standarisiert auf palycyamidem 3 mg
400 mg D-alpha-Tocopherol Konzentrat
200 mg β-Hydroxyäthylrutosid
150 mg Sojaöl
50 mg Sojalecithin (2 x 1 Kapsel täglich)

**BEISPIEL 90**

Kombipackung enthält:
Kapsel A enthaltend
50 mg Weißdorn Fruct. Crataegus Sicc. standarisiert auf playcyamidem 2,5 mg

300 mg Vitamin E
130 mg Rüböl
40 mg Hydriertes Sojabohnenöl
10 mg Sojalecithin
Kapsel B enthaltend
1,5 mg Dihydroergotoxinmethansulphonat-Gemische
50 mg Sojaöl
250 mg Sojalecithin

**BEISPIEL 91**

Kapsel enthaltend
40 mg Fruct. Crataegus sicc. stand. auf palycymidem 2,5mg
400 mg Dl-alpha-Tocopherolacetat
70 mg Cinnarizin
100 mg Sojaöl
50 mg Hydriertes Sojaöl
30 mg Sojalecithin

**BEISPIEL 92**

Kombipackung aus:
Kapsel A enthaltend
60 mg Fruct. Crataegus sicc. stand. auf palycyamidem 3 mg
150 mg Sojabohenöl
50 mg Sojalecithin
Kapsel B enthaltend
400 mg Dl-alpha-Tocopherolacetat
100 mg Bencyclanfumarat
100 mg Sojaöl

**BEISPIEL 93**

Kombipackung enthält:
Kapsel enthaltend
400 mg Vitamin E
400 mg Sojalecithin 45%
70 mg Sojabohnenöl
Kapsel enthaltend
60 mg Fruct. Crataegus Sicc. stand. auf playcyaniden 3 mg
300 mg ß-Hydroxyäthylrutosid
100 mg Sojaöl

Derartige Produkte welche hohe Anteile an Lecithin haben, beeinflußen den Lipid- Cholesterinwechsel sehr positiv, welche bei Herzerkrankungen von großer Bedeutung sind. Die Einnahme der beiden Kapseln erfolgt gleichzeitig 2 bis 3 mal Täglich.

**BEISPIEL 94**

Kapsel enthaltend
30 mg Fruct. Crataegus sicc. stand. auf palycyaniden 1,5 mg
150 mg Troxerutin
200 mg Vitamin E
300 mg Sojalecithin,
100 mg Sojaöl

Es wird bei dieser Kapsel empfohlen 2 x 2 Kapsel bis 3 x 2 Kapseln Täglich einzünehmen, um die Lipid und Cholesterinstoffwechsel günstig zu beeinflußen.

**BEISPIEL 95**

Kombipackung enthält:
Kapsel enthaltend
60 mg Fruct. Crataegus sicc. stand. auf palycyaniden 3 mg
200 mg Vitamin E
100 mg Sojaöl
Kapsel enthaltend
300 mg Extrct. Hippocastani
200 mg Vitamin E
150 mg Sojaöl
Es wird hier empfohlen von dieser Kapsel 2 x 2 bzw. 3 x 2 Kapseln Täglich einzunehmen.

**BEISPIEL 96**

Kapsel aus:
400 mg Peracetam
400 mg Dl-alpha-Tocopherolacetat
150 mg Sojaöl
50 mg Sojalecithin

**BEISPIEL 97**

Kapsel enthaltend
400 mg D-alpha-Tocopherol Konzentrat
200 mg $\beta$-Hydroxyäthylrutosid
150 mg Sojaöl
50 mg Sojalecithin

**BEISPIEL 98**

Kombipackung enthält:
Kapsel A enthaltend
400 mg Peracetam
400 mg Vitamin E
130 mg Rüböl
40 mg Hydriertes Sojabohnenöl
10 mg Sojalecithin
Kapsel B enthaltend
1,5 mg Dihydroergotoxinmethansulphonat-Gemische
50 mg Sojaöl
100 mg Sojalecithin

**BEISPIEL 99**

Kapsel enthaltend
350 mg Peracetam
400 mg Dl-alpha-Tocopherolacetat
70 mg Cinnarizin
100 mg Sojaöl
50 mg Hydriertes Sojaöl
30 mg Sojalecithin

**BEISPIEL 100**

Kombipackung aus:
Kapsel A enthaltend
600 mg Peracetam
150 mg Sojabohnenöl
50 mg Sojalecithin

Kapsel B enthaltend
400 mg DI-alpha-Tocopherolacetat
250 mg Nicotinsäure
100 mg Sojaöl

**Patentansprüche**

1. Verwendung von Vitamin E in Mengen von 150 bis 1000 i. E. pro Darreichungsform, gegebenenfalls in Gegenwart von durchblutungsfördernden und/oder gefäßerweiternden Mitteln, Vitamin A, Vitamin C, Vitaminen der B-Reihe, Schmerzmitteln, Antiphlogistika, Antirheumamitteln, Emulgatoren und/oder üblichen Hilfsstoffen zur Herstellung eines Arzneimittels zur Verbesserung der Eigenschaften des Blutes, wobei das Arzneimittel in Darreichungsformen aus der Gruppe Kapseln, Tabletten, Dragees und Suppositorien vorliegt.

2. Verwendung nach Anspruch 1 mit Mengen an Vitamin E im Bereich von 300 bis 600 i. E., vorzugsweise von 400 bis 600 i. E., pro Darreichungsform.

3. Verwendung nach einem der Ansprüche 1 oder 2, wobei das Arzneimittel in der Darreichungsform von Weichgelatine-Kapseln vorliegt.

4. Verwendung nach einem oder mehreren der Ansprüche 1 bis 3, wobei Vitamin E neben einem oder mehreren durchblutungsfördernden Mitteln aus der Gruppe Cinnarizin, Vincamin, Bamethansulfat, Extr. Hippocastani, Weißdorn, Peracetam, Nicergolin, Buflomedil, Flunarizin, Bencyclanhydrogenfumarat, Dihydroergotoxinmethansulfonat, β-Pyridylcarbinol, Ginkoflavonglykoside, β-Hydroxyethylrutosid, Calciumdobesilat und Pentoxyphyllin verwendet wird.

5. Verwendung nach einem oder mehreren der Ansprüche 1 bis 4, wobei Vitamin E neben einem oder mehreren Schmerzmitteln aus der Gruppe Acetylsalicylsäure, Dichlofenac, Pyrazolon und dessen Derivate, Phenacetin und Paracetamol und dessen Derivate verwendet wird.

6. Verwendung nach einem oder mehreren der Ansprüche 1 bis 5, wobei Vitamin E neben 20 bis 70 Gew.-% Lecithin, bezogen auf die Inhaltsstoffe, in der Darreichungsform einer Kapsel verwendet wird.

7. Verwendung nach einem oder mehreren der Ansprüche 1 bis 6, wobei 300 bis 600 i. E., vorzugsweise 400 bis 500 i. E., Vitamin E in Kombination mit 200 bis 600 mg Nicotinsäure und/oder 150 bis 800 mg, vorzugsweise 400 bis 600 mg, Pentoxyphyllin pro Darreichungsform verwendet werden.

8. Verwendung nach einem oder mehreren der Ansprüche 1 bis 7, wobei Vitamin E neben 15 bis 90 mg, vorzugsweise 20 bis 70 mg, Weißdornwirkstoffe pro Darreichungsform verwendet werden.

9. Verwendung nach einem oder mehreren der Ansprüche 1 bis 8, wobei Vitamin E neben 300 bis 700 mg Calciumdobesilat pro Darreichungsform verwendet werden.

10. Verwendung nach einem oder mehreren der Ansprüche 1 bis 9, wobei die Eigenschaften des Blutes seine Fließeigenschaften und/oder seine die Immunabwehr betreffenden Eigenschaften und/oder seine die Beeinflussung abnormaler Vergrößerungen von Zellen oder von Tumoren betreffenden Eigenschaften sind.

**Claims**

1. Use of vitamin E in an amount of from 150 to 1,000 International Units (I.U.) per dosage form, optionally in the presence of blood circulation-promoting agents and/or vasodilators, vitamin A, vitamin C, vitamins of the B-series, analgesics, antiphlogistics, antirheumatics, emulsifiers and/or conventional auxiliary materials for the preparation of a medicament for improving the properties of the blood, the medicament being formulated into dosage forms from the group of capsules, tablets, dragées and suppositories.

2. The use according to claim 1, wherein the amounts of vitamin E are within the range of from 300 to 600 I.U., and preferably within the range of from 400 to 600 I.U., per dosage form.

3. The use according to any of claims 1 or 2, wherein the medicament is present in the dosage form of soft gelatin capsules.

4. The use according to any one or more of claims 1 to 3, wherein vitamin E is used in addition to one or more blood circulation-promoting agents from the group of Cinnarizine, Vincamine, Bamethan sulfate, Extract. hippocastani, hawthorn, Peracetam, Nicergoline, Buflomedil, Flunarizine, Bencyclan hydrogenfumarate, dihydroergotoxine methanesulfonate, beta-pyridylcarbinol, Gingko flavoglycosides, β-hydroxyethyl rutoside, calcium dobesilate and Pentoxyfylline.

5. The use according to any one or more of claims 1 to 4, wherein vitamin E is used in addition to one or more analgesics from group of acetylsalicylic acid, Dichlofenac, pyrazolone and its derivatives, Phenacetin and Paracetamol and its derivatives.

6. The use according to any one or more of claims 1 to 5, wherein vitamin E is used in addition to from 20 to 70% by weight of lecithine in the dosage form of a capsule.

7. The use according to any one or more of claims 1 to 6, wherein vitamin E is used in amounts of from 300 to 600 I.U., and preferably of from 400 to 500 I.U., in combination with from 200 to 600 mg of nicotinic acid and/or from 150 to 800 mg, and preferably from 400 to 600 mg, of Pentoxyphylline per dosage unit.

8. The use according to any one or more of claims 1 to 7, wherein vitamin E is used in addition to from 15 to 90 mg, and preferably from 20 to 70 mg, of active hawthorn ingredients per dosage form.

9. The use according to any one or more of claims 1 to 8, wherein vitamin E is used in addition to from 300 to 700 mg of calcium dobesilate per dosage form.

10. The use according to any one or more of claims 1 to 9, wherein the properties of the blood are the flow properties thereof and/or the properties thereof relating to immune defense and/or the properties relating to influencing abnormal enlargements of cells or of tumours.

**Revendications**

1. Utilisation de la vitamine E à raison de 150 à 1.000 U.I. par forme d'administration, éventuellement en présence de substances favorisant la circulation du sang et/ou vasodilatatrices, de vitamine A, de vitamine C, de vitamines de la série B, d'analgésiques, de substances antiphlogistiques, d'anrhumatismaux, d'émulsifiants et/ou d'adjuvants usuels pour la préparation d'un médicament destiné à améliorer les propriétés du sang, caractérisée en ce que le médicament se présente sous des formes d'administration du groupe comprenant des capsules, des comprimés, des dragées et des suppositoires.

2. Utilisation selon la revendication 1, avec des quantités de vitamine E comprises dans la plage de 300 à 600 U.I., de préférence de 400 à 600 U.I. par forme d'administration.

3. Utilisation selon l'une des revendications 1 et 2, caractérisée en ce que le médicament se présente sous la forme d'administration de capsules de gélatine molle.

4. Utilisation selon l'une ou plusieurs des revendications 1 à 3, caractérisée en ce qu'on utilise la vitamine E en plus d'une ou plusieurs substances favorisant la circulation du sang, choisie(s) dans le groupe comprenant la cinnarizine, la vincamine, le sulfate de baméthan, l'extract. hippocastani, l'aubépine, le peracétame, la nicergoline, le buflomédil, la flunarizine, l'hydrogénofumarate de bencyclane, le méthanesulfonate de dihydroergotoxine, le β-pyridylcarbinol, les glucosides de gingkoflavone, le β-hydroxyéthylrutoside, le dobésilate de calcium et la pentoxyphylline.

5. Utilisation selon l'une ou plusieurs des revendications 1 à 4, caractérisée en ce qu'on utilise la vitamine E en plus d'un ou plusieurs analgésiques choisi(s) dans le groupe comprenant l'acide acétylsalicylique,

EP 0 204 987 B1

le diclofénac, la pyrazolone et ses dérivés, la phénacétine et le paracétamol et ses dérivés.

6. Utilisation selon l'une ou plusieurs des revendications 1 à 5, caractérisée en ce qu'on utilise la vitamine E en plus de 20 à 70 % en poids de lécithine, par rapport aux substances contenues, sous la forme d'administration d'une capsule.

7. Utilisation selon l'une ou plusieurs des revendications 1 à 6, caractérisée en ce qu'on utilise 300 à 600 U.I., de préférence 400 à 500 U.I., de vitamine E, combinées à 200 à 600 mg d'acide nicotinique et/ou 150 à 800 mg, de préférence 400 à 600 mg, de pentoxyphylline par forme d'administration.

8. Utilisation selon l'une ou plusieurs des revendications 1 à 7, caractérisée en ce qu'on utilise la vitamine E en plus de 15 à 90 mg, de préférence de 20 à 70 mg, de principes actifs d'aubépine par forme d'administration.

9. Utilisation selon l'une ou plusieurs des revendications 1 à 8, caractérisée en ce qu'on utilise la vitamine E en plus de 300 à 700 mg de dobésilate de calcium par forme d'administration.

10. Utilisation selon l'une ou plusieurs des revendications 1 à 9, caractérisée en ce que les propriétés du sang sont les propriétés concernant ses caractéristiques d'écoulement et/ou ses caractéristiques concernant la défense immunitaire et/ou ses caractéristiques concernant l'influence de proliférations anormales de cellules ou de tumeurs.